# EUROPÄISCHE PATENTSCHRIFT

(11) **EP 2 182 916 B1**
(45) Veröffentlichungstag und Bekanntmachung des Hinweises auf die Patenterteilung: **12.12.2012**
(21) Anmeldenummer: 08784831.3
(22) Anmeldetag: 17.07.2008
(51) Int. Cl.: A61K 8/365, A61K 8/26, A61K 8/28, A61Q 15/00, A61K 8/86, A61K 8/20, A61K 8/04

(54) **KOSMETISCHE ODER DERMATOLOGISCHE FORMULIERUNG ENTHALTEND REINE D- ODER L-MANDELSÄURE**
COSMETIC OR DERMATOLOGICAL FORMULATION CONTAINING D OR L MANDELIC ACID
FORMULATION COSMÉTIQUE OU DERMATOLOGIQUE CONTENANT DE L'ACIDE MANDÉLIQUE D OU L

(30) Priorität: 24.07.2007 DE 102007035741
(43) Veröffentlichungstag der Anmeldung: 12.05.2010
(73) Patentinhaber: Beiersdorf AG, 20253 Hamburg (DE)
(72) Erfinder: BIEL, Stefan, 21077 Hamburg (DE); WEINERT, Katrin, 22764 Hamburg (DE)
(74) Vertreter: Hartmann, Jost
(86) Internationale Anmeldenummer: PCT/EP2008/005849
(87) Internationale Veröffentlichungsnummer: WO 2009/012925

(56) Entgegenhaltungen:
- EP-A1- 1 787 629
- WO-A1-2005/105027
- WO-A2-03/039505
- NESCI A. ET AL: "Control of Aspergillus growth and aflatoxin production using antioxidants at different conditions of water activity and pH" JOURNAL OF APPLIED MICROBIOLOGY, Bd. 95, Nr. 2, 11. Juli 2003 (2003-07-11), Seiten 279-287, XP002517216 The Society for Applied Microbiology
- TARA E. GOTTSCHALCK ET AL: "International Cosmetic Ingredient Dictionary and Handbook, 11th edition, 4 Volume set" Januar 2006 (2006-01), THE COSMETIC, TOILETRY, AND FRAGRANCE ASSOCIATION , USA , XP002517970 ISBN 1-882621-34-4 Monograph 2642 Monograph 2888

## Beschreibung

Die Erfindung betrifft eine kosmetische oder dermatologische Formulierung umfassend reine D- oder L-Mandelsäure, Antitranspirantien, Wasser und ggf. Hilfsstoffe sowie deren verbesserte Stabilisierung.

In der DE 102005017032 werden Mikroemulsionen enthaltend einen Antitranspirantwirkstoff, Mandelsäure und polyethoxylierte O/W-Emulgatoren offenbart.

In der WO 2005105026 werden transparente kosmetische und/oder dermatologische Formulierung offenbart, die mindestens einen Antitranspirant-Wirkstoff und/oder Deodorant-Wirkstoff, mindestens eine α-Hydroxycarbonsäure und Wasser umfassen. Als Hydroxysäure ist Mandelsäure bevorzugt zu wählen.
Die vorliegende Anmeldung umfasst den Offenbarungsgehalt der Anmeldung WO 2005105026 vollumfänglich.

Mithilfe der Mandelsäure-Geltechnologie, wie sie in der WO 2005105026 beschrieben ist, ist es erstmals möglich transparente, insbesondere Roll-On-Systeme herzustellen, in die aufgrund der Fließgrenze dieser Formulierungen beispielsweise auch visuelle Effekte wie z.B. Schwebeteilchen, Beads eingearbeitet werden können.

Allerdings zeigt sich, dass diese Mandelsäuresysteme unter besonderen Lagerbedingungen (Lichtlagerung, Lagerung bei 40°C) mitunter nicht lange stabil bleiben, ohne dass Ausfällungen oder Kristallisationen auftreten können.

Wünschenswert ist es daher Mandelsäuregele, insbesondere transparente und insbesondere als Antitranspirant- oder Deodorantprodukte, bereit zu stellen, die eine verbesserte Stabilisierung bei Sonnenbestrahlung und/oder erhöhter Temperatur aufweisen.
Die vorliegende Erfindung umfasst eine kosmetische Formulierung umfassen mindestens einen Antitranspirantwirkstoff, reine Enantiomere in D- oder L-Form der Mandelsäure, Wasser und ggf. einen Hilfsstoff.
Überraschenderweise wird durch den Einsatz der reinen Enantiomere in D- oder L-Form der Mandelsäure die zur Vergelung notwendige Mandelsäure-Konzentration um bis zu 50% reduziert, so dass in der Folge auch weniger Mandelsäure ausfallen/kristallisieren kann. Beispielsweise wird bei der Vergelung von einer wäßrigen Lösung mit 10 Gew.% ACH (Aluminiumchlorohydrat) mit 1 Gew.% reiner D- oder L-Mandelsäure eine gleiche Gelstärke erreicht wie bei Einsatz einer Menge von 2 Gew.% D/L-Mandelsäure.
Die erfindungsgemäße Formulierung lässt sich zu einer viskosen bis pastösen Formulierung gelieren und ermöglicht die Bereitstellung einer transparenten und wenig klebrigen kosmetischen Zubereitung, insbesondere eine Antitranspirant- bzw. Deodorantzubereitung wie in der WO 2005105026 beschrieben. Zum Unterschied werden durch den Einsatz der reinen Enantiomere der Mandelsäure (D- oder L-Form) die zeitweilig auftretenden Probleme durch Ausfällungen erfindungsgemäß minimiert.

Die Hydroxyphenylessigsäure oder auch Phenylglykolsäure mit der Formel H₅C₆ -CH(OH) -COOH, C₈H₈O₃ ist bekannt unter dem Namen Mandelsäure. Die Mandelsäure ist gut löslich in Wasser, Alkohol, Ether u. 2-Propanol. Synthetisch erhält man die (±)-, auch D- oder L-Mandelsäure aus Benzaldehyd und Blausäure über das α-Hydroxynitril (Cyanohydrin) und dessen saure Hydrolyse entsprechend Abbildung 1: 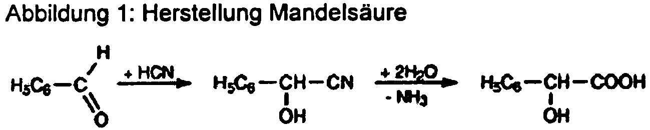 Mittels der Mandelsäure, läßt sich überraschenderweise eine AT- bzw. Deodorantzubereitung aber auch eine beliebige kosmetische Zubereitung herstellen, die die vorteilhaften Eigenschaften, wie Transparenz, geringe Klebrigkeit, Parfumbestandteile und darüber hinaus auch die Einstellung einer bestimmten Fließgrenze der Zubereitung ermöglicht. Des weiteren zieht die erfindungsgemäße Formulierung sehr schnell ohne Rückstände zu hinterlassen in die Haut ein.
Darüber hinaus trägt Mandelsäure durch ihre keratinolytischen Eigenschaften zur Regeneration der Achselhaut bei und wirkt im sauren Milieu der Achsel bakteriostatisch. Aufgrund dieser Eigenschaften ist Mandelsäure hervorragend auch als aktive Komponente in kosmetischen und insbesondere Deo/AT-Produkten geeignet.
Die Fließgrenze oder Fließpunkt ist eine Bezeichnung für die kleinste Schubspannung, oberhalb derer ein plastischer Stoff sich rheologisch wie eine Flüssigkeit verhält (DIN 1342-1: 1983-10). Die Bestimmung der Fließgrenze erfolgt durch Aufnahme einer Fließkurve (DIN 53019: 1980-05; DIN 53214: 1982-02). Der erhaltene Wert hängt stark von der Zeitskala (Belastungsrate) ab, die der Messung zugrunde liegt. Dies ist unabhängig davon, ob die Messung mit einem schubspannungs- oder drehzahlgesteuerten Viskosimeter erfolgt. Kurze Zeitskalen (schnelle Belastungen) ergeben in der Regel höhere Werte für die Fließgrenze. Eine zu hohe fließgrenze kann Ursache von Verlaufstörungen sein. Andererseits lässt sich mit geeignet bemessener Fließgrenze die Neigung der flüssigen Formulierung zum Ablaufen unterdrücken.

Die erfindungsgemäße Zubereitung liegt daher vorteilhaft als Gel- bzw. Hydrogel vor und weist ein Fließgrenze auf, wodurch die Ausbringung und Applikation gegenüber den Zubereitungen aus dem Stand der Technik verbessert ist.

Erfindungsgemäß wird anstelle einer Mischung aus D- und L- Mandelsäure nun reine D - oder L-Mandelsäure verwendet und man gelangt zu einer kosmetischen Zubereitung mit gleichen Charakteristika wie die in der WO 2005105026 beschriebenen. Die reinen D-bzw. L-Enantiomere können käuflich erworben werden, z.B. als R-(-)- oder S-(+)-Mandelsäure bei Merck Darmstadt.
Jedoch ist nun die Menge an D- bzw. L-Mandelsäure erfindungsgemäß verringert, was wiederum eine bessere Stabilisierung und eine geringe Anfälligkeit gegen äußere Einflüsse, wie Sonnenbestrahlung oder Temperaturschwankungen bewirkt. Vorzugsweise beträgt der Gesamtanteil an Mandelsäure (D- oder L-Form) erfindungsgemäß maximal bis zu 2 Gew.%, vorteilhaft maximal 1,4 Gew.%, bezogen auf die Gesamtmasse einer Zubereitung.

Die erfindungsgemäße Kombination aus AT-Wirkstoff, D- oder L-Mandelsäure, ggf. Hilfsstoff und Wasser ermöglicht über einen einzigartigen Verdickungsmechanismus die Herstelltung einer insbesondere transparenten kosmetischen Zubereitung. Der Anwender hat somit erstmalig eine wasserklare und dennoch überaus wirksame Zubereitung zur Hand. Die erfindungsgemäße Zubereitung ist in Gelform bequem zu applizieren und weist eine angenehmes Hautgefühl aufgrund der fehlenden Klebrigkeit auf.

Als Antitranspirantwirkstoff lassen sich vorteilhaft saure Aluminium- und/oder Aluminium/Zirkoniumsalze in wässriger Lösung einarbeiten. Hierbei beziehen sich die beschriebenen Konzentrationsbereiche auf die so genannten Aktivgehalte der Antitranspirant-Komplexe: bei den Aluminium-Verbindungen auf wasserfreie Komplexe, bei den Aluminium/Zirkonium-Verbindungen auf wasser- und pufferfreie Komplexe. Als Puffer wird hier üblicherweise Glycin verwendet.

Die nachfolgende Auflistung vorteilhaft einzusetzender Antitranspirant-Wirker soll in keiner Weise einschränkend sein:
Aluminium-Salze (der empirischen Summenformel [Al₂(OH)ₘClₙ], wobei m+n=6):
   - Aluminiumchlorhydrat [Al₂(OH)₅Cl] x H₂O
      Standard Al-Komplexe: Locron L, Locron LIC, Locron LIF (Clariant), Chlorhydrol (Reheis), ACH-303 (Summit), Aloxicoll L (Giulini).
      Aktivierte Al-Komplexe: Reach 501 (Reheis), Aloxicoll 51 L
   - Aluminiumsesquichlorhydrat [Al₂(OH)₄,₅Cl_{1,5}] x H₂O
      Standard Al-Komplexe: Aloxicoll 31 L (Giulini), Westchlor 186 (Westwood Chemicals)
      Aktivierte Al-Komplexe: Reach 301 (Reheis)
   - Aluminiumdichlorhydrat [Al₂(OH)₄Cl₂] x H₂O

### Aluminium-Zirkonium-Salze:

- Aluminium/Zirkonium Trichlorhydrex Glycin [Al₄Zr(OH)₁₃Cl₃] x H₂O x Gly
   Standard Al/Zr-Komplexe: Rezal 33GC (Reheis), AZG-7164 (Summit)
- Aluminium/Zirkonium Tetrachlorhydrex Glycin [Al₄Zr(OH)₁₂Cl₄] x H₂O x Gly
   Standard Al/Zr-Komplexe: Rezal 36, Rezal 36G, Rezal 36 GC (Reheis), AZG-368 (Summit), Zirkonal L435G (Giulini), Westchlor ZR 35 BX5, Westchlor ZR 41 (Westwood Chemicals)
- Aluminium/Zirkonium Pentachlorhydrex Glycin [Al₈Zr(OH)₂₃Cl₅] x H₂O x Gly
   Standard Al/Zr-Komplexe: Rezal 67 (Reheis), Zirkonal L540, Zirkonal L530 PG (Giulini), Westchlor ZR 80B (Westwood Chemicals)
- Aluminium/Zirkonium Octachlorhydrex Glycin [Al₈Zr(OH)₂₀Cl₈] x H₂O x Gly: Westchlor ZR 82B

Ebenso vorteilhaft können aber auch Glycin-freie Aluminium/Zirkonium-Salze eingesetzt werden.

Die Antitranspirant-Wirkstoffe werden in den erfindungsgemäßen Formulierungen in einer Menge von 1 bis 35 Gew.%, vorzugsweise von 1 bis 20 Gew. %, eingesetzt.

Vorteilhaft wird der AT-Wirkstoff aus den Aluminiumsalzen, insbesondere Aluminiumchlorohydrat, aktiviert oder unaktiviert, gewählt.

Vorteilhaft können erfindungsgemäßen Zubereitungen auch Desodorantien zugesetzt werden. Den üblichen kosmetischen Desodorantien liegen unterschiedliche Wirkprinzipien zugrunde.
Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluss selbst wird dadurch nicht beeinflusst, im Idealfalle wird nur die mikrobielle Zersetzung des Schweißes zeitweilig gestoppt. Auch die Kombination von Adstringentien mit antimikrobiell wirksamen Stoffen in ein und derselben Zusammensetzung ist gebräuchlich.
Alle für Desodorantien gängigen Wirkstoffe können vorteilhaft genutzt werden, beispielsweise Geruchsüberdecker wie die gängigen Parfüme oder deren Parfümbestandteile, Geruchsabsorber, beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden.
Bevorzugt umfasst die erfindungsgemäße Zubereitung ein oder mehrere Parfüme und/oder deren Bestandteile.
Die Menge der Desodorantien (eine oder mehrere Verbindungen) in den Zubereitungen beträgt vorzugsweise 0,01 bis 10 Gew.%, bevorzugt 0,05 bis 5 Gew.% bezogen auf das Gesamtgewicht der Zubereitung.

Bevorzugt können den Zubereitungen Hilfsstoffe zugesetzt werden. Diese als Hilfsstoffe benannten Verbindungen werden gewählt aus
a.)der Gruppe der polyethoxylierte hydrogenierte castor Öle, PEG-40 hydrogenated castor oil, PEG-60 hydrogenated castor oil
b.)Ethylendinitrilotetraessigsäure (EDTA) und/oder ein Salz davon und/oder
c.) Calcium-dinatrium-ethylendiamintetraacetat,
d.) phenolische Antioxidantien, wie beispielsweise die Handelsprodukte Tinogard TS, Tinogard TT, Tinogard NOA der Fa.Ciba und/oder
e.) ein oder mehrere Disulfite, insbesondere Natriumdisulfit.

PEG-40 hydrogenated castor oil ist als Handelprodukt unter dem Namen Solutor, Eumulgin oder Fancol erhältlich.

PEG-40 hydrogenated castor oil, lässt sich verteilhaft in Kombination mit Parfum einsetzen.

Parfum ist für viele Kosmetika ein essentieller Bestandteil, aber er führt häufig zu Problemen und Instabilitäten.
Mit dem oder den erfindungsgemäßen Hilfsstoffen, wie zuvor angeführt, insbesondere dem Hilfsstoff oder Lösevermittler Solutor (PEG-40), lassen sich Parfume erfindungsgemäß in das Mandelsäure Gel einarbeiten ohne das es trüb oder instabil wird. D.h. es ist weiterhin eine vorteilhafte Transparenz und Stabilität von Mandelsäure Gelen, wie sie in der WO 2005105026 beschrieben sind, gegeben.

Gebräuchliche Anteile an Hilfsstoffen liegen vorteilhaft im Bereich von 1 bis 10 Gew.%. insbesondere etwa 4 bis 7 Gew.%, bevorzugt im Bereich von 5 bis 6 Gew.%, jeweils bezogen auf die Gesamtmasse der Zubereitung.

Als bevorzugte Hilfsstoffe können daher Lösevermittler, insbesondere PEG-40 HYDROGENATED CASTOR OIL (Warenbez.: Solutor, Eumulgin, Fancol, Cremophor), die insbesondere in Kombination mit Parfüm und/oder deren Bestandteilen, gewählt werden. Ausgenommen als Hilfsstoffe sind die in der DE 102005017032 als Emulgatoren genannten Polyethylenglycolglycerinfettsäureester, Polyethylenglycol(15)glyceryllaurat und/oder Glycerylmonostearate.
Als Hilfsstoffe können des Weiteren vorteilhaft Disulfite eingesetzt werden, insbesondere Natriumdisulfit.

Der Zusatz von Natriumdisulfit führt zur Vermeidung von Verfärbungsreaktionen und/oder Bildung von Fehlgeruch.
Fehlgerüche und Verfärbungen bilden sich durch ungewollte Oxidationsprozesse. Angeregt werden diese z.B. durch Licht und Wärme. Im Falle der Mandelsäure kann mitunter während der Lagerung Benzaldehyd entstehen. Durch Zusatz der Hilfsstoffe, insbesondere von Disulfiten, insbesondere Natriumdisulfit oder Kombinationen von EDTA und Natriumdisulfit, werden diese Oxidationsprozesse unterdrückt und somit Verfärbung und Fehlgeruchsausbildung minimiert. Der Anteil an Disulfiten liegt vorteilhaft bei etwa 0,1 bis 0,2 Gew.%.

Als weitere Ausführungsform kann als Hilfsstoff EDTA, Ethylendinitrilotetraessigsäure, und/oder ein Salz davon und/oder Calcium-dinatrium-ethylendiamintetraacetat eingesetzt werden.

Die Hilfsstoffgruppe der phenolische Antioxidantien umfasst bevorzugt Pentaerythrityl Tetra-di-t-butyl Hydroxyhydrocinnamate (Handelsname Tinogard TT, Ciba), Tetrabutyl Ethylidinebisphenol (Handelsname Tinogard NOA, Ciba) oder Octadecyl Di-t-butyl-4-hydroxyhydrocinnamate (Handelsname Tinogard TS, Ciba).
Vorteilhaft ist neben der Wahl reiner D- oder L-Mandelsäure auch der Zusatz ein oder mehrerer Hilfststoffe gewählt aus der Gruppe der Substanzen a. bis e.

Bevorzugt ist insbesondere auch die Kombination zweier Hilfsstoffe, z.B. EDTA und PEG-40 hydrogenated castor oil oder Natriumdisulfit und EDTA.

Dem erfindungsgemäßen System kann weiterhin zusätzlich
- Co-Verdicker, wie beispielsweise Polyvinylpyrrolidone (z.B. Luviskol 30, BASF),
- Polymere enthaltend Maleinsäure oder Maleinsäureanhydrid (z.B. Gantrez S- oder Gantrez AN-Typen, ISP International Specialty Products) und/oder
- hydrophob modifzierte Cellulosen (Natrosol 250 HHX Pharm, Hercules)
- Salze, insbesondere bivalente Salze wie beispielsweise Magnesiumchlorid oder Calciumchlorid
in Konzentrationen von 0,1% bis 5%, allein oder in Kombination zugeben werden.

Auch diese Co-Verdicker, Polymere bzw. Cellulose und Salze haben einen zusätzlich stabilisierenden Einfluss auf die erfindungsgemäßen Mandelsäuregele.

Der Zusatz von Magnesiumchlorid und Calciumchlorid führt beispielsweise zu einer Erhöhung der Scherstabilität der Mandelsäuregele.

Die bivalenten Neutralsalze Magnesiumchlorid und Calciumchlorid führen zu einer Scherstabilität der erfindungsgemäßen Zubereitungen indem ohne den Zusatz weiße Eintrübungen entstehen können, wenn die Formulierung z.B. stärker geschüttelt wird, die Formel aus einem Roller appliziert wird oder man ggf. zu spät abfüllt. Mit dem Zusatz der Salze passiert das nicht mehr.

Es ist erfindungsgemäß möglich durch den Zusatz ein oder mehrerer Hilfsstoffe die Stabilität von Mandelsäure enthaltenden kosmetischen Zubereitungen zu verbessern. Als Stabilitätsverbesserung ist dabei insbesondere das Vermeiden von Ausfällungen, Eintrübungen oder Kristallisationen, die Stabilisierung bei Sonnenbestrahlung und/oder erhöhter Temperatur, die Vermeidung von Verfärbungsreaktionen und/oder Bildung von Fehigeruch sowie die Erhöhung der Scherstabilität der Mandelsäurezubereitungen zu verstehen. Letzteres ist insbesondere auch nur durch den Zusatz von Salzen möglich.

Die erfindungsgemäßen kosmetischen und dermatologischen Zubereitungen können kosmetische Zusatzstoffe enthalten, wie sie üblicherweise in solchen Zubereitungen verwendet werden, z.B. Konservierungsmittel, Bakterizide, UV-Filter, Antioxidantien, wasserlösliche Vitamine, Mineralstoffe, suspendierte Festkörperpartikel, Parfüme, Substanzen zum Verhindern des Schäumens, Farbstoffe, Pigmente, die eine färbende Wirkung haben, Verdickungsmittel, anfeuchtende und/oder feuchthaltende Substanzen oder andere übliche Bestandteile einer kosmetischen oder dermatologischen Formulierung wie Alkohole, Polyole, Polymere, Schaumstabilisatoren oder Silikonderivate.
Diese Zusatzstoffe sind von den genannten erfindungsgemäßen Hilfsstoffen zu unterscheiden.

Vorteilhaft werden zur Herstellung erfindungsgemäßer Zubereitungen zunächst ein oder mehrere Hilfsstoffe und ggf. Parfum zusammen gegeben. Der Hilfsstoff und Parfum werden anschließend zu annähernd gleichen Teilen auf die wässrigen Lösungen von D-oder L-Mandelsäure und ACH verteilt, bevor man diese zwecks Vergelung zusammengibt.

Die Angaben der Beispiele beziehen sich auf Gewichtsprozent bezogen auf die Gesamtmasse der Zubereitung.

### Beispiele

### Transparente Gelstick-Systeme

| Ingredients | 1 | 2 |
|---|---|---|
| (L)-Mandelsäure | 1,1 | 0,8 |
| Aluminiumchlorohydrate (50%) | 20,0 | 10,0 |
| Trisodium EDTA | 1,0 | 1,0 |
| PEG-40- Hydrogenated Castor Oil | 5,0 | 5,0 |
| Parfüm | q.s. | q.s. |
| Wasser | ad 100% | ad 100% |

| Ingredients | 3 | 4 |
|---|---|---|
| (L)-Mandelsäure | 1,2 | 1,3 |
| Aluminiumchlorohydrate (50%) | 20,0 | 20,0 |
| Trisodium EDTA | 1,0 | 1,0 |
| PEG-40- Hydrogenated Castor Oil | 5,0 | 5,0 |
| PVP K30 | 0,5 | 1,0 |
| Parfüm | q.s. | q.s. |
| Wasser | ad 100% | ad 100% |

| Ingredients | 5 | 6 |
|---|---|---|
| (D)-Mandelsäure | 1,1 | 0,8 |
| Aluminiumchlorohydrate (50%) | 20,0 | 10,0 |
| Trisodium EDTA | 1,0 | 1,0 |
| PEG-40- Hydrogenated Castor Oil | 5,0 | 5,0 |
| Parfüm | q.s. | q.s. |
| Wasser | ad 100% | ad 100% |

| Ingredients | 7 | 8 |
|---|---|---|
| (L)-Mandelsäure | 1,1 | 0,8 |
| Aluminiumchlorohydrate (50%) | 20,0 | 10,0 |
| Wasser | ad 100% | ad 100% |

### Transparente Gel-Roll-on-Systeme:

| Ingredients | 9 | 10 |
|---|---|---|
| (L)-Mandelsäure | 0,6 | 0,4 |
| Aluminiumchlorohydrate (50%) | 20,0 | 10,0 |
| Trisodium EDTA | 1,0 | 1,0 |
| PEG-40- Hydrogenated Castor Oil | 5,0 | 5,0 |
| Parfüm | q.s. | q.s. |
| Wasser | ad 100% | ad 100% |

| Ingredients | 11 | 12 |
|---|---|---|
| (L)-Mandelsäure | 0,7 | 0,8 |
| Aluminiumchlorohydrate (50%) | 20,0 | 20,0 |
| Trisodium EDTA | 1,0 | 1,0 |
| PEG-40- Hydrogenated Castor Oil | 5,0 | 5,0 |
| PVP K30 | 0,5 | 1,0 |
| Parfüm | q.s. | q.s. |
| Wasser | ad 100% | ad 100% |

| Ingredients | 13 | 14 |
|---|---|---|
| (D)-Mandelsäure | 0,6 | 0,8 |
| Aluminiumchlorohydrate (50%) | 20,0 | 20,0 |
| Trisodium EDTA | 1,0 | 1,0 |
| PEG-40- Hydrogenated Castor Oil | 5,0 | 5,0 |
| PVP K30 | - | 1,0 |
| Parfüm | q.s. | q.s. |
| Wasser | ad 100% | ad 100% |

| Ingredients | 15 | 16 |
|---|---|---|
| (D)-Mandelsäure | 0,6 | 0,8 |
| Aluminiumchlorohydrate (50%) | 20,0 | 20,0 |
| Trisodium EDTA | 1,0 | 1,0 |
| PEG-40- Hydrogenated Castor Oil | - | 5,0 |
| Calciumchlorid | 0,9 | 1,0 |
| PVP K30 | - | 1,0 |
| Parfüm | q.s. | q.s. |
| Wasser | ad 100% | ad 100% |

## Patentansprüche

1. Kosmetische und/oder dermatologische Formulierung umfassend mindestens einen Antitranspirant-Wirkstoff. Mandelsäure und Wasser, **dadurch gekennzeichnet, dass** reine D- oder L-Mandelsaure gewählt wird.

2. Formulierung nach Anspruch 1 umfassend mindestens einen Hilfsstoff, gewählt aus der Gruppe der polyethoxylierten hydrogenierten Rizinusöle, PEG-40 hydrogenated castor oil, PEG-60 hydrogenated castor oil und/oder Ethylendinitrilotetraessigsäure (EDTA) und/oder ein Salz davon und/oder Calcium-dinatrium-ethylendiamintetraacetat und/oder phenolische Antioxidantien und/oder Disulfite.

3. Formulierung nach Anspruch 2, **dadurch gekennzeichnet, dass** der oder die Hilfsstoffe gewählt werden aus der Gruppe der Pentaerythrityl Tetra-di-t-butyl Hydroxyhydrocinnamate, Tetrabutyl Ethylidinebisphenol, Octadecyl Di-t-butyl-4-hydroxyhydrocinnamate und/oder Natriumdisulfit.

4. Formulierung nach einem der vorstehenden Ansprüche umfassend Magnesium- und/oder Calciumchlorid.

5. Formulierung nach einem der vorstehenden Ansprüche umfassend ein oder mehrere Parfume und/oder deren Bestandteile.

6. Formulierung nach einem der vorstehenden Ansprüche, **dadurch gekennzeichnet, dass** als Hilfsstoffe PEG-40 hydrogenated castor oil und EDTA gewählt werden.

7. Formulierung nach einem der vorstehenden Ansprüche, **dadurch gekennzeichnet, dass** der Antitranspirant-Wirkstoff aus der Gruppe der Aluminium-Salze, bevorzugt Aluminium-Chlorohydrat oder Aluminium-Zirkonium-Salze, gewählt wird.

8. Verwendung von Magnesium- und/oder Calciumchlorid zur Scherstabilisierung von reinen D- oder L-Mandelsäure enthaltenden Zubereitungen,

## Claims

1. Cosmetic and/or dermatological formulation comprising at least one antiperspirant active ingredient, mandelic acid and water, **characterized in that** pure D- or L-mandelic acid is selected.

2. Formulation according to Claim 1, comprising at least one auxiliary selected from the group of polyethoxylated hydrogenated castor oils, PEG-40 hydrogenated castor oil, PEG-60 hydrogenated castor oil and/or ethylenedinitrilotetraacetic acid (EDTA) and/or a salt thereof and/or calcium disodium ethylenediaminetetraacetate and/or phenolic antioxidants and/or disulphites.

3. Formulation according to Claim 2, **characterized in that** the auxiliary or the auxiliaries are selected from the group of Pentaerythrityl Tetra-dit-butyl- Hydroxyhydrocinnamate, Tetrabutyl Ethylidinebisphenol, Octadecyl Di-t-butyl-4-hydroxyhydrocinnamate and/or sodium disulphite.

4. Formulation according to one of the preceding claims, comprising magnesium chloride and/or calcium chloride.

5. Formulation according to one of the preceding claims, comprising one or more perfumes and/or constituents thereof.

6. Formulation according to one of the preceding claims, **characterized in that** the auxiliaries selected are PEG-40 hydrogenated castor oil and EDTA.

7. Formulation according to one of the preceding claims, **characterized in that** the antiperspirant active ingredient is selected from the group of aluminium salts, preferably aluminium chlorohydrate or aluminium zirconium salts.

8. Use of magnesium chloride and/or calcium chloride for the shear stabilization of preparations comprising pure D- or L-mandelic acid.

## Revendications

1. Formulation cosmétique et/ou dermatologique comprenant au moins une substance active antitranspirante, de l'acide mandélique et de l'eau, **caractérisée en ce qu'**on choisit de l'acide D-mandélique ou L-mandélique pur.

2. Formulation selon la revendication 1, comprenant au moins un adjuvant choisi dans le groupe des huiles de ricin hydrogénées polyéthoxylées, l'huile de ricin hydrogénée par PEG-40, l'huile de ricin hydrogénée par PEG-60 et/ou l'acide éthylènedinitrilotétraacétique (EDTA) et/ou un sel de celui-ci et/ou l'éthylènediaminetétraacétate de calcium et disodique et/ou des antioxydants phénoliques et/ou des bisulfites.

3. Formulation selon la revendication 2, **caractérisée en ce que** le ou les adjuvants sont choisis dans le groupe pentaérythrityl-tétra-di-t-butyl-hydroxyhydrocinnamate, tétrabutyl-éthylidinebisphénol, octadécyl-di-t-butyl-4-hydroxyhydrocinnamate et/ou bisulfite de sodium.

4. Formulation selon l'une quelconque des revendications précédentes, comprenant du chlorure de magnésium et/ou de calcium.

5. Formulation selon l'une quelconque des revendications précédentes, comprenant un ou plusieurs parfums et/ou leurs constituants.

6. Formulation selon l'une quelconque des revendications précédentes, **caractérisée en ce qu'**on choisit, comme adjuvants, l'huile de castor hydrogénée par PEG-40 et l'EDTA.

7. Formulation selon l'une quelconque des revendications précédentes, **caractérisée en ce que** la substance active antitranspirante est choisie dans le groupe des sels d'aluminium, de préférence le chlorhydrate d'aluminium ou les sels d'aluminium et de zirconium.

8. Utilisation de chlorure de magnésium et/ou de calcium pour la stabilisation au cisaillement de compositions contenant de l'acide D-mandélique ou L-mandélique pur.
